# EUROPÄISCHE PATENTSCHRIFT

(11) **EP 1 165 014 B1**
(45) Veröffentlichungstag und Bekanntmachung des Hinweises auf die Patenterteilung: **25.08.2004**
(21) Anmeldenummer: 00917025.9
(22) Anmeldetag: 31.03.2000
(51) Int. Cl.: A61K 6/083, C04B 28/28

(54) **POLYELEKTROLYTZEMENT**
POLYELECTROLYTE CEMENT
CIMENT AUX POLYELECTROLYTES

(30) Priorität: 01.04.1999 DE 19914975
(43) Veröffentlichungstag der Anmeldung: 02.01.2002
(73) Patentinhaber: 3M ESPE AG, 82229 Seefeld (DE)
(72) Erfinder: BRAUN, Peter, D-86899 Landsberg (DE); GLASER, Jürgen, D-82237 Steinebach (DE); NIRSCHL, Hermann, D-82229 Seefeld (DE); STEFAN, Klaus-Peter, D-82229 Seefeld (DE)
(74) Vertreter: Brem, Roland
(86) Internationale Anmeldenummer: PCT/EP2000/002855
(87) Internationale Veröffentlichungsnummer: WO 2000/059452

(56) Entgegenhaltungen:
- EP-A- 0 219 058
- WO-A-92/21632
- GB-A- 2 173 184
- GB-A- 2 173 207

## Beschreibung

Die Erfindung betrifft einen ein- oder mehrkomponentigen Polyelektrolytzement, der mindestens zwei Reaktionspartner enthält, (a) eine Metallkationen-freisetzende Verbindung und (b) einen oder mehrere in den Festzustand überführbare Polyelektrolyten, wobei mindestens einer der Polyelektrolyte zumindest teilweise wasserlöslich ist, und wobei mindestens ein Teil der Reaktionspartner (a) und / oder (b) mit einem organischen Oberflächenbeschichtungsmittel überzögen ist. Die Erfindung betrifft ferner ein aus mindestens einem Teil der in fester Form vorliegenden Rezepturbestandteilen des Polyelektrolytzements erhältliches Granulat, wobei im Sinne einer autogenen Granulierung zumindest ein Teil des Reaktionspartners (b) als wesentliches Granuliermittel dient und das Granulat im Kontakt mit den flüssigen Rezepturbestandteilen wieder in das Primärkorn zerfällt.

Ferner betrifft die Erfindung Verfahren zur Herstellung des Granulats sowie die Verwendung des Polyelektrolytzements als Dentalmaterial.

Unter Polyelektrolyten werden im Sinne der vorliegenden Erfindung Polymere mit ionisch dissoziierbaren Gruppen verstanden, die Bestandteil oder Substituent der Polymerkette sein können und deren Zahl so groß ist, daß die Polymeren zumindest in ihrer partiell dissoziierten Form wenigstens teilweise wasserlöslich sind. Unter Polyelektrolytzementen werden im Sinne der vorliegenden Erfindung Materialien verstanden, die einen Polyelektrolyten enthalten. Insbesondere sollten diese Polyelektrolyten mit der Metallkationen-freisetzenden Verbindung im Sinne einer Chelatbildungsreaktion, besonders bevorzugt einer Säure-Base-Reaktion/Neutralisationsreaktion reagieren können. Diese Reaktion wird als Abbindereaktion oder kurz Abbindung bezeichnet. Neben dieser Abbindung kann bei Zusatz von polymerisierbaren Verbindungen und für die Polymerisation dieser Verbindungen geeigneten Initiatoren zusätzlich eine Polymerisationsreaktion ablaufen.

Derartige Polyelektrolytzemente werden z.B. durch Reaktion einer Polyalkensäure, insbesondere einer Polyacrylsäure, mit Zinkoxid bzw. einem Metalikationenfreisetzenden, sog. basischen Glaspulver in Gegenwart von Wasser erhalten. Diese Zemente sind als Polycarboxylatzemente [D.C. Smith, Biomaterials 19, 467-478 (1998)] seit 1967 bzw. als (konventionelle) Glasionomerzemente (Glaspolyalkenoatzemente, GIZ) [A. D. Wilson, B. E. Kent, DE 20 61 513] seit 1969 bekannt. Polyelektrolytzemente, die zusätzlich polymerisierbare Verbindungen sowie geeignete Initiatoren enthalten, sind beispielsweise kunststoffmodifizierte Glasionomerzemente [s. beispielsweise R. Mathis, I. L. Ferracane, J. Dent. Res. 66, 113 (Abstract 51) (1987)] oder Compomere [s. beispielsweise EP 219 058].

Die o.g. Polyelektrolytzemente sind als zweikomponentige Paste-Paste-Systeme und einkomponentige Pastesysteme formulierbar. Üblicherweise werden die o.g. Polyelektrolytzemente jedoch als Pulver-Flüssigkeits-Systeme formuliert. Dabei kann sich der Polyelektrolyt entweder in der Flüssigkeit befinden, oder er ist als Feststoff dem Pulver beigemischt. Auch Mischformen, bei denen Teile des Polyelektrolyten im Pulver und Teile in der Flüssigkeit enthalten sind, sind bekannt [s. beispielsweise GB -A-17880-72, DE-A-2319715]. Feststoffmischungen, in denen zumindest ein Teil des Polyelektrolyten neben einer Metallkationen-freisetzenden Verbindung vorliegt, werden als "trockene Pulvermischung" bezeichnet.

Der Zusatz des Polyelektrolyten als Feststoff zum Pulver ist beispielsweise dann von Vorteil, wenn zusätzliche Verarbeitungszeit durch das Lösen des Polyelektrolyten gewonnen werden soll oder wenn die vollständige Polyelektrolytmenge in der Lösung zu einer zu hohen und daher nicht mehr geeigneten Viskosität und Verarbeitbarkeit führt.

Nachteil der trockenen Pulvermischung ist, daß bei Zutritt von Feuchtigkeit, z.B. Luftfeuchtigkeit, bei der Lagerung des Produktes bis zum Gebrauch eine Reaktion zwischen den beiden Reaktionspartnern, d.h. der Metallkationen-freisetzenden Verbindung und dem Polyelektrolyt, einsetzt, die zu einer Verlangsamung der Zementabbindung führt. Damit ist eine zuverlässige Anwendung des Polyelektrolytzements nicht mehr gewährleistet, weil die Abbindung des Materials in Abhängigkeit mit der Lagerdauer zunimmt.

Dies spielt insbesondere bei den Handmischvarianten dieser Polyelektrolytzemente eine wichtige Rolle, weil diese Produkte für den kostenbewußten Anwender so konzipiert sind, daß mehrere Applikationen mit dem abgefüllten Material durchführbar sind. Dafür wird die trockene Pulvermischung in kleinen Gläschen dargereicht, in welche bei jeder Entnahme Luftfeuchtigkeit gelangt, die eine Verlangsamung der Abbindung verursacht. Außerdem wird die Anmischbarkeit mit der Zeit schwieriger, weil sich aufgrund der in Gegenwart von Luftfeuchte oberflächlich ablaufenden Reaktion der Metallkationen-freisetzenden Verbindung mit dem Polyelektrolyten Agglomerate erhöhter Festigkeit bilden können, deren Zerscherung nur durch einen erhöhten Energieeintrag beim Mischen erreicht werden kann.

Eine über die Lagerzeit konstante Abbindung kann durch Verhinderung von Feuchtigkeitszutritt zu der trockenen Pulvermischung erreicht werden. Dies ist nur durch einen erhöhten Verpackungsaufwand realisierbar: Beispielsweise werden derartige Polyelektrolytzemente, die in einer für eine Einmalapplikation entwickelten Mischkapsel angeboten werden, in Aluminiumverbundfolien gegebenenfalls mit einem zusätzlichen Trockenkissen verblistert. Diese Maßnahme hat zwar den gewünschten Stabilisierungseffekt auf die Abbindung, ist jedoch mit deutlich erhöhten Kosten bei der Herstellung und damit für den Verbraucher verbunden. Zudem sinkt im Rahmen eines ständig steigenden Umweltbewußtseins des Verbrauchers die Akzeptanz eines aufwendig verpackten Produktes.

Auch bei der Herstellung und Abfüllung der trockenen Pulvermischung sind spezielle Maßnahmen zu ergreifen, um den Kontakt mit Luftfeuchte möglichst gering zu halten, da sonst eine initiale Schädigung der trockenen Pulvermischung auftreten und die Abfüllung der trockenen Pulvermischung erschwert werden kann, was zu einem erhöhten Wartungsaufwand führt.

Eine andere Möglichkeit, prinzipiell Stoffe vor Umwelteinflüssen zu schützen, besteht darin, die zu schützenden Stoffe mit einem Überzug zu versehen.

Aus der pharmazeutischen Industrie sind organische Überzugsmassen bekannt, die bei der Tablettenherstellung verwendet werden [H. P. Fiedler, Lexikon der Hilfsstoffe für Pharmazie, Kosmetik und angrenzende Gebiete, Editio Cantor Verlag Aulendorf, 4. Aufl., 1996, S. 1498-1500]. Diese Überzugsmassen sind je nach Anwendungsgebiet der Tablette säurelöslich (Löslichkeit im Magen), alkalilöslich (Darmlöslichkeit) oder wasserlöslich. Typische Vertreter dieser sehr breit verwendeten Tablettenüberzugsmassen sind die Eudragit® -Typen der Firma Röhm. Eudragit® L (säureresistent) ist ein säurefunktionelles Polymer, während Eudragit® E (säurelöslich) Aminogruppen aufweist [H. P. Fiedler, Lexikon der Hilfsstoffe für Pharmazie, Kosmetik und angrenzende Gebiete, Editio Cantor Verlag Aulendorf, 4. Aufl., 1996, S. 596-598]. Ebenfalls aminofuktionell, aber wasserlöslich ist das Produkt Copolymer 845 der Firma ISP, welches tertiäre Amino- und Pyrrolidongruppen aufweist. Auch Überzugsmassen auf Basis von Cellulosederivaten (z. B. OPADRY® 11, der Firma Colorcon oder Sepifilm, der Firma Seppic) sind bekannt. Copolymere, die auch Polysaccharide enthalten, wie z. B. Surelease (von Colorcon) werden ebenfalls für diesen Zweck eingesetzt (s. Produktkataloge der einzelnen Anbieter).

Um mit diesen Überzugsmassen Feuchteresistenz zu erzielen, geben die Hersteller Empfehlungen zu den dafür erforderlichen Filmstärken ab. Beispielsweise empfielt die Firma Röhm zur Herstellung von feuchtigkeitsresistenten Tablettenüberzügen Filmstärken von ca. 10 µm, was etwa einem Milligramm Überzugsmasse pro cm² entspricht (Eudragit® -Produktkatalog der Firma Röhm). Um auf den feingemahlenen Bestandteilen des Pulvers eines Polyelektrolytzementen (spezifische Oberfläche von etwa 3 m² pro Gramm) die empfohlene Filmstärke zu erreichen, müßten diese daher mit ca. 30 Gramm Überzugsmasse pro Gramm Pulver beschichtet werden.

Dies stellt einen wesentlichen Eingriff in die Zusammensetzung des Polyelektrolytzements dar. Besonders kritisch ist dabei zu sehen, daß es sich bei den zur Oberflächenbeschichtung genannten Materialien um im Sinne einer Polyelektrolytzementreaktion nichtreaktive Zusätze handelt. Bereits frühere Versuche haben immer wieder gezeigt, daß nichtreaktive Zusätze i.a. bei Polyelektrolytzementen in Konzentrationen von wenigen Prozent bereits zu einer deutlichen Verschlechterung der Eigenschaften, insbesondere der mechanischen Werte, wie Druck- oder Biegefestigkeit, führen.

Die DE 3610844 und DE 3610845 beschreiben Oberflächenbeschichtungsmittel für Dentalzemente auf Calciumaluminatbasis. Diese härten im Gegensatz zu Polyelektrolytzementen durch eine Hydratisierung und nicht durch ein Chelatbildung oder Neutralisation aus. Da diese Zemente im basischen Medium, ohne Beteiligung einer Säure aushärten, sind die Anforderungen an die Säureresistenz der Beschichtungsmittel mit den Anforderungen, die an einen Polyelektrolytzement gestellt werden, nicht vergleichbar. Die in diesen Schriften zur Oberflächenbeschichtung eingesetzten Polymere sind nur wasserlöslich und werden auch in der wässrigen Reaktionslösung, die zur Hydratisierung verwendet wird, in bis zu 10%iger Konzentration als Verdicker eingesetzt.

Es ist daher die Aufgabe der vorliegenden Erfindung, einen leicht produzier-, abfüllund anmischbaren Polyelektrolytzement zur Verfügung zu stellen, der gegenüber Feuchtigkeit so stabil ist, daß sich im Rahmen einer üblichen Haltbarkeit weder seine Beschaffenheit noch seine Abbindung ändert. Wenn möglich, ohne daß hierfür gesonderte packmitteltechnische Maßnahmen ergriffen werden müssten, und ohne daß die mechanischen Werte, wie Druck- oder Biegefestigkeit wesentlich verringert werden.

Erfindungsgemäß wird diese Aufgabe durch einen ein- oder mehrkomponentigen Polyelektrolytzement und/oder ein Granulat gelöst, wie es in den Ansprüchen beschrieben wird.

Überraschenderweise lassen sich durch eine oberflächliche Beschichtung zumindest eines Teils der Metallkationen-freisetzenden Verbindung und/oder der Polyelektrolyte im Gegensatz zu den im Stand der Technik bekannten Polyelektrolytzementen gegenüber Luftfeuchtekontakt so stabile Polyelektrolyzemente erhalten, daß sich im Rahmen einer üblichen Haltbarkeit weder ihre Abbindung noch ihre Beschaffenheit ändert, so daß ihre Anwendbarkeit nicht negativ beeinflußt wird und die mechanischen Werte denen der im Stand der Technik bekannten Polyelektrolyzemente vergleichbar sind.

Überraschender Weise reduziert sich zudem die Klebrigkeit des Polyelektrolytzements am Bearbeitungsinstrument, und der Abbindeübergang wird vorteilhafterweise verkürzt.

Kennzeichnend für die Oberflächenbeschichtungsmittel im Sinne der Erfindung ist, daß es sich um filmbildende Materialien handelt, die aus einer Lösung auf die Oberfläche eines festen Kerns (reaktive Komponente des Polyelektrolytzements) abgeschieden werden, danach diesen Kern zumindest teilweise bedecken ohne einen festen chemischen Verbund mit dem Kern einzugehen. Im Rahmen der Polyelektrolytzementreaktion sind die Oberflächenbeschichtungsmittel vorzugsweise von dem Kern wieder ablösbar, während der Lagerung schützen sie den Kein jedoch vor Feuchteeinfluß.

Reaktive Materialien, die einen chemischen Verbund mit der Oberfläche der Reaktivkomponente des Polyelektrolytzemntes bilden (z.B. Silanisierungsmittel, wie sie in der DE 3941629, DE 19526224 oder DE 19605272 beschrieben werden) sind nicht Oberflächenbeschichtungsmittel im Sinne der Erfindung.

Bevorzugt werden filmbildende Materialien, die aufgrund ihrer toxikologischen Unbedenklichkeit bereits als Überzugsmittel in der pharmazeutischen Industrie eingesetzt werden (s.o.). Besonders bevorzugt werden polymere, filmbildende Materialien, insbesonders diejenigen mit einer Molmasse größer 10000. Besonders geeignet sind dabei Polymere, die im wässrig sauren Medium eine ausreichend hohe Löslichkeit aufweisen, so daß sie schnell genug die Reaktivkomponente freigeben, und auf diese Weise keine nennenswerte Verzögerung der Abbindereaktion auftritt. Bevorzugt sollte die Verzögerung 1 Minute, besonders bevorzugt 30 Sekunden nicht überschreiten.

Diese Forderung steht im Widerspruch zur Forderung eines wirksamen Feuchtigkeitsschutzes. Überraschenderweise zeigte sich, daß an sich bekannte, filmbildende Materialien, die bei Filmdragees in einer Dicke von 40 - 200 µm eingesetzt werden, diese Forderungen auch erfüllen, wenn sie auf die Reaktivkomponente eines Polyelektrolytzements in einer Konzentration kleiner 3% bevorzugt kleiner 2% besonders bevorzugt kleiner 1% (bezogen auf das Gewicht des Polyelektrolytzements) aufgebracht werden.

Aufgrund der hohen spezifischen Oberfläche der Komponenten, die im Bereich 0,2 bis 10, bevorzugt 0,5 bis 5, besonders bevorzugt 1 bis 3 m²/g liegen, entspricht dies nur einer Filmdicke von einigen nm und ist damit um einen Faktor 1000 bis 50000 geringer als in der pharmazeutischen Industrie üblich. Um die hohe Löslichkeit zu gewährleisten werden magensaftlösliche Tablettenüberzugsmittel bevorzugt, besonders bevorzugt werden dabei Polymere, die aminofunktionelle Comonomere enthalten. Diese Polymere können sowohl als solche als auch in einer neutralisierten oder teilneutralisierten Form eingesetzt werden. Zur Neutralisation können dabei alle Säuren eingesetzt werden. Bevorzugt werden jedoch organische Säuren, besonders bevorzugt Carbonsäuren, insbesondere Säuren, die in Polyelektrolytzementen bekanntermaßen eingesetzt werden können, wie Hydroxycarbonsäuren, -im Speziellen Weinsäure oder Citronensäure.

Überaschenderweise hat sich gezeigt, daß deutlich geringere Beschichtungen als von der pharmazeutischen Industrie für organische Überzugsmassen, die-bei der Tablettenherstellung verwendet werden, empfohlen werden, genügen, um die erfindungsgemäße Aufgabe zu lösen. Bereits Beschichtungen im Bereich 0,01 bis 3 Gew.-%, bevorzugt 0,1 bis 2% und ganz besonders bevorzugt 0,2 bis 1,5 Gew.-%, führen zu stabilen Systemen. In diesem Konzentrationsbereich sind die zur Beschichtung eingesetzten Materialien für die übrigen Zementeigenschaften unkritisch. Die genannten Gew.-%-Angaben beziehen sich auf das Gesamtgewicht des oberflächenbeschichteten Materials

In den Zeichnungen zeigen
- Abb. 1:: eine graphische Darstellung der Verarbeitungs- und Lagerzeiten von mit Eudragit® beschichteten Gläsern (Beispiele 2 bis 7) im Vergleich zu einem unbeschichteten Glas (Beispiel 1),
- Abb. 2:: eine graphische Darstellung der Verarbeitungs- und Lagerzeiten von mit anderen Beschichtungsmitteln überzogenen Gläsern (Beispiele 8 bis 10) im Vergleich zu einem unbeschichteten Glas (Beispiel 1),
- Abb. 3:: eine graphische Darstellung der Verarbeitungs- und Lagerzeiten von mit Eudragit® beschichtetem Zinkoxid (Beispiel 12) im Vergleich zu unbeschichtem Zinkoxid (Beispiel 11).

Polyelektrolytzemente können ein- oder mehrkomponentig formuliert werden. Das bedeutet, daß die Bestandteile des Polyelektrolytzementes in einer oder mehreren Komponenten des Zementes separat verpackt zur Verfügung gestellt werden. Dies ist dann notwendig, wenn einzelne Reaktionspartner getrennt voneinander gelagert werden müssen, weil die sonst einsetzende Reaktion zwischen ihnen die Lagerstabilität des Polyelektrolytzementes negativ beeinflußt. Beispielsweise müssen die drei Reaktionspartner in Glasionomerzementen (GIZ), das basische Glaspulver, die Polyalkensäure und Wasser, so voneinander getrennt aufbewahrt werden, daß keine Reaktion einsetzt. Glasionomere werden deshalb mindestens zweikomponentig angeboten: Eine Komponente kann das basische Glaspulver und die Polyalkensäure und die zweite Komponente das Wasser enthalten. Die Polyalkensäure kann sich auch partiell oder vollständig im Wasser befinden. Nach dem Vermischen der beiden Komponenten startet die Abbindung. Nur wenn die beiden Reaktionspartner (a) und (b) zumindest teilweise in derselben Komponente vorliegen, ist mit einem Lagerstabilitätsproblem aufgrund einer Reaktion zwischen diesen beiden Reaktionspartnern zu rechnen. Dann ist es erfindungsgemäß vorteilhaft, wenn mindestens ein Teil der in derselben Komponente befindlichen Reaktionspartner (a) und/oder (b) mit einem organischen Oberflächenbeschichtungsmittel oberflächlich beschichtet ist.

Damit die Abbindung ablaufen kann, ist Wasser als Reaktionspartner erforderlich. Die benötigte Menge kann dabei in Abhängigkeit der Reaktionspartner beträchtlich variieren. Es ist auch möglich, daß der erfindungsgemäße Polyelektrolytzement selbst kein Wasser enthält, sondern das zur Reaktion erforderliche Wasser aus der Umgebung, z.B. dem Mund des Patienten, stammt. Dies ist beispielsweise bei der Materialklasse der o.g. Compomere der Fall. Da die Abbindung zwischen den beiden Reaktionspartnern (a) und (b) ohne Wasser nicht abläuft, können beispielsweise lichthärtende Compomere auch einkomponentig formuliert werden. Die Reaktion zwischen der Metallkationen-freisetzenden Verbindung und dem Reaktionspartner (b) ist hier ebenso wie die benötige Wassermenge (aus dem Mund des Patienten) von untergeordneter Bedeutung im Vergleich zur Polymerisationsreaktion, die hier durch den Zusatz von polymerisierbaren Verbindungen und für die Polymerisation dieser Verbindungen geeigneten Initiatoren zusätzlich abläuft.

Wasser befindet sich in den erfindungsgemäßen Polyelektrolytzementen zu 0 - 30 Gew.-%, vorzugsweise 0-25 Gew.-%. Dabei enthalten die Compomere i.a. kein oder keine nennenswerten Mengen Wasser. Kunststoffmodifizierte Glasionomerzemente enthalten bevorzugt 3-12 und ganz besonders bevorzugt 5 - 10 Gew.-% Wasser. Glasionomerzemente und Polycarboxylatzemente enthalten besonders bevorzugt 5 - 25 Gew.-% Wasser. Beispielsweise enthält ein GIZ 5 - 20 Gew.-%, vorzugsweise 8 - 15 Gew.-%, Wasser. Die genannten Gew.-%-Angaben beziehen sich, sofern nichts anderes angegeben ist, jeweils auf das Gesamtgewicht des Polyelektrolytzements.

Unter der Metallkationen-freisetzenden Verbindung sollen im Sinne der vorliegenden Erfindung alle Stoffe verstanden werden, die in der Lage sind, Metallkationen freizusetzen, die dann mit dem Reaktionspartner (b) im Sinne einer Chelatbildungsreaktion reagieren können. Dabei sollen die freigesetzten Kationen vorzugsweise mehrwertig sein, besonders bevorzugt 2- und 3-wertig. Derartige Verbindungen werden für den Polyelektrolytzement in pulvriger Form in einer Korngröße hergestellt, wie sie für Polyelektrolytzemente üblich ist [s. beispielsweise DE-A-2061513].

Beispiele für die Metallkationen-freisetzende Verbindung sind Metallsalze, insbesondere Metalloxide und Metallhydroxide, besonders bevorzugt aus der Gruppe der Erdalkalimetalle, wie z.B. CaO, MgO, Ca(OH)₂, Mg(OH)₂, und ZnO, bestimmte feinverteilte Metalle, wie z.B. feinverteiltes Zink, etc. (US-A-3,028,247), und basische Glaspulver, die vor allem durch ihren Anteil an 2- und 3-wertigen lonen, wie z.B. Ca²⁺, Sr²⁺, Ba²⁺, La²⁺, Y³⁺ Al³⁺, in Gegenwart von Wasser zur Reaktion mit dem Reaktionspartner (b) geeignet sind (s. beispielsweise DE-A-2061513, EP-A-0023013, EP-A-02 41 277). Weitere Beispiele sind Metallkationen-freisetzende Silikate, wie z.B. Schichtsilikate, wie z.B. Montmorillonite, Bentonite, oder Calciumsilikate, Zirkonsilikate, Natriumatuminiumsilikate, und Zeolithe, einschließlich der Molekularsiebe.

Der erfindungsgemäße Polyelektrolytzement enthält die Metallkationen-freisetzende Verbindung vorzugsweise zu einem Anteil von 15 bis 85 Gew.-%, besonders bevorzugt 18 bis 80 Gew.-% und ganz besonders bevorzugt 50 bis 70 Gew.-%, bezogen auf die Gesamtzusammensetzung.

Der nach der vorliegenden Erfindung verwendete Polyelektrolyt (b) ist ein Polymer mit ionisch dissoziierbaren Gruppen, die Substituenten der Polymerkette sein können und deren Zahl so groß ist, daß die Polymeren zumindest in ihrer (partiell) dissoziierten Form wenigstens teilweise wasserlöslich sind. Hierfür sind insbesondere Substituenten wie z.B. -COOH, -OH, -PO(OH)₂, -OPO(OH)₂, -SO₂(OH), geeignet. Besonders bevorzugt sind organische Polysäuren (DE-A-20 61 513), wie z.B. Polymere und Copolymere der Acrylsäure, Methacrylsäure (EP-A-0 024 056), Itaconsäure, Maleinsäure, Citraconsäure, Phosphonsäure (EP-A-0 340 016; GB-A-22 91 060). Daneben können beim Vorliegen mehrerer Polyelektrolyte auch wasserunlösliche Polyelektrolyte im Polyelektrolytzement vorliegen. Voraussetzung ist lediglich, daß mindestens einer der Polyelektrolyte nach obiger Definition wenigstens teilweise wasserlöslich sein muß.

Unter "in den Festzustand überführbar" soll im Sinne der vorliegenden Erfindung verstanden werden, daß der Polyelektrolyt entweder per se bei Raumtemperatur ein Feststoff oder aber zumindest in seiner partiell oder vollständig dissoziierten Form ein Feststoff ist.

Die Polyelektrolyten sollen mit der Metallkationen-freisetzenden Pulverkomponente im Sinne einer Chelatbildungsreaktion, bevorzugt einer Säure-Base-Reaktion/Neutralisationsreaktion, reagieren können.

Weitere, nicht in den Festzustand überführbare Polyelektrolyte können sich ebenfalls im Polyelektrolytzement befinden, jedoch getrennt von der Metallkationen-freisetzenden Verbindung in einer anderen Komponente des erfindungsgemäßen, dann mehrkomponentigen Polyelektrolytzementes.

Der erfindungsgemäße Polyelektrolytzement enthält den wenigstens teilweise wasserlöslichen, in den Festzustand überführbaren Polyelektrolyten vorzugsweise zu einem Anteil von 0,5 bis 30 Gew.-%, besonders bevorzugt 2 bis 25 Gew.-% und ganz besonders bevorzugt 5 bis 20 Gew.-%.

Erfindungsgemäß wird mindestens ein Teil der Reaktionspartner (a) und / oder (b) mit einem organischen Oberflächenbeschichtungsmittel oberflächlich beschichtet. Dabei können die in der pharmazeutischen Industrie bekannten organischen Überzugsmassen verwendet werden, die auch bei der Tablettenherstellung Einsatz finden (s.o.).

Die Oberflächenbeschichtungen können mit sehr unterschiedlichen Materialien und Methoden durchgeführt werden. Bevorzugt werden polymere Verbindungen verwendet. Beispielsweise finden Zuckerlösungen, Polyacrylate und Methacrylate, Lösungen auf Basis von Gummi arabicum, Gelatine, Methylcellulose, andere Cellulosederivate, oder Polyethylenglykole Verwendung [s. Ullmanns Encyklopädie der technischen Chemie, Verlag Chemie, Weinheim, 4. Auflage, 1979, 18-155ff; H. P. Fiedler, Lexikon der Hilfsstoffe für Pharmazie, Kosmetik und angrenzende Gebiete, Editio Cantor Verlag Aulendorf, 4. Aufl., 1996, S. 1498-1500]. Besonders bevorzugt handelt es sich bei dem organischen Oberflächenbeschichtungsmittel um ein zumindest teilweise säurelösliches Oberflächenbeschichtungsmittel. Ganz besonders bevorzugt handelt es sich bei dem organischen Oberflächenbeschichtungsmittel um sogenannte magensaftlösliche Oberflächenbeschichtungsmittel einschließlich ihrer Salze. Besonders geeignet als Beschichtungsmaterialien sind z. B. Polymere mit Aminogruppen (wie z. B. Eudragit® E der Firma Röhm), die sowohl in ihrer aminischen Form, als auch in einer, z.B. mit Weinsäure, neutralisierten Form einsetzbar sind (s.u.). Auch polymere Materialien auf Polysacharidbasis, wie z. B. OPADRY® , oder auf Basis von Cellulosederivaten, wie z. B. Surelease oder Sepifilm, führen zu gleich guten Ergebnissen.

Wichtige Vertreter dieser Produktklassen sind in der folgenden Tabelle wiedergegeben. Diese Materialien seien beispielhaft für Oberflächenbeschichtungsmittel im Sinne der Erfindung genannt.

**Tablelle:**

| Filmbildner als Oberflächenbeschichtungsmittel | | | |
|---|---|---|---|
| **Typ** | **Filmbildner** | **Molmasse** | **Lit.** |
| Natürliche Filmbildner | Schellack (Exkret der indischen Gummilackschildlaus) | Ca. 1000 | 1,2,3, 4 |
| | Mastix (Pistazienharz) | | 1,3 |
| | Sandarac (Harz von Callitris quadrivalvis) | | 1,3 |
| | Tolubalsam (Harz von Myroxylon balsamum) | | 1,3 |
| | Dammarharz (Katzenaugenharz, Harz des Dammar-baums) | | 1,3 |
| | Benzoeharz (asiat. Baumharz) | | 1,3 |
| | Keratin (Hornstoff z.B. aus Fedem) | 40.000 - 70.000 | 1,2,3 |
| | Maisin (Zein) (Proteine aus Maissamen) | 10.000 - 22.000 | 1,3 |
| | Gummi Arabicum | | 2 |
| | Gelatine | | 2 |
| Halbsynthetische Filmbildner | Mit Formaldehyd behandelte Gelatine | | 1,3 |
| | Salol (Acetaladehyd-Phenol-Condensat) | | 1,3 |
| Cellulosederivate | Celluloseacetatphtalat | 40.000 | 2,2a, 4 |
| | Hydroxyethylcellulose | | 2,4 |
| | Methylcellulose | 20.000 - 150.000 | 4 |
| | Hydroxypropylmethylcellulose | 10.000 - 150.000 | 2a,4 |
| | Hydroxypropylcellulose | 60.000 - 1.200.000 | 4 |
| | Natrium-Carboxymethylcellulose | 80.000 - 600.000 | 4 |
| | Ethylcellulose (z.B. Surelease - Colorcon) | | 4,7 |
| | Hydroxypropylmethylcellulosephthalat | 20.000 | 4 |
| Poly(meth)acrylate | Copolymere aus Methacrylsäureestern und aminofunktionellen Methacrylaten (z.B. Eudragit E - Röhm, Copolymer 845 - ISP) | 150.000 (Eudragit E) 800.000 (Eudragit E30D) | 2,4,5, 6 |
| | Copolymere aus (Meth)acrylsäure und Methylmethacrylat (z.B. Eurdagit L, Eudragit S, Eudragit RL, Eudragit RS - Röhm) | 135.000 (Eudragit L bzw. S) 150.000 (Eudragit RL bzw. RS) 250.000 (Eudragit L30D) | 2,4,5 |
| Vinylpolymere | Polyvinylpyrrolidon | 10.000 - 350.000 | 2a,4 |
| | Polyvinylacetatphthalat | 25.000 - 40.000 | 4 |
| Gemische | z.B. Hydroxypropyl- und Hydroxyporpyl-methylcellulose (z.B. Spifilm -Seppic) | | 8 |
| | z.B. Hydroxypropylcellulose und Polysaccharide (z.B. Opadry II - Colorcon) | | 7 |
| 1. F. Gstirner, Grundstoffe und Verfahren der Arzneibereitung, Ferdinand Enke Verlag, Stuttgart, 1960, S. 529 ff 2. P.H. List et. al., Arzneiformenlehre, 4. Aufl., Wissenschaftliche Verlagsgesellschaft mbH, Stuttgart; 1985, S. 127 ff 2a. ibid. S.540 3. Römpp Chemie Lexikon, 9. Aufl. Georg Thieme Verlag, Stuttgart, New York, 1995 4. K. H. Bauer, K.-H. Frömming, C. Führer, Pharmazeutische Technologie, 3. Aufl., Georg Thieme Verlag, Stuttgart, New York, 1991 S. 314 ff 5. H.P. Fiedler Lexikon der Hilfsstoffe, Editio Cantor Verlag Aulendorf, 1996 6. ISP, Prospekt 7. Colorcon, Prospekt 8. Seppic, Prospekt | | | |

Da die meisten in der Literatur beschriebenen Oberflächenbeschichtungsmittel nur in organischen Lösungsmitteln für die Oberflächenbeschichtung ausreichend löslich sind, müssen die beim Umgang mit organischen Lösungsmitteln üblichen Vorsichtsmaßnahmen für den Explosionsschutz ergriffen werden. Dies läßt sich dadurch vereinfachen, indem die Oberflächenbeschichtungsmittel in eine wasserlösliche Form gebracht werden. Dies kann durch übliche Maßnahmen, wie z. B. durch die Einführung polarer oder ionischer Gruppen in das Oberflächenbeschichtungsmittel, erreicht werden. Die Neutralisation von sauren oder basischen Gruppen ist hierfür besonders geeignet. Beispielsweise läßt sich Eudragit® E in ein für die Oberflächenbeschichtung ausreichend wasserlösliches Eudragit® -E-Salz, z.B. sein Tartrat, überführen, so daß bei der Oberflächenbeschichtung auf Maßnahmen für den Explosionsschutz verzichtet werden kann, weil nun Wasser als Lösungsmittel bei der Oberflächenbeschichtung eingesetzt werden kann.

Die Oberflächenbeschichtung kann gemäß den in der Literatur beschriebenen Verfahren durchgeführt werden. Ohne die Erfindung darauf einzuschränken, seien im Folgenden beispielhaft einige Verfahren kurz angesprochen.

Zunächst werden die zu beschichtenden Feststoffe zusammen mit dem in einem geeigneten Lösungsmittel gelösten Oberflächenbeschichtungsmittel zu einer breiigen Konsistenz angeteigt und dann in einem Aggregat unter starker Scherung homogen vermischt. Als Aggregate kommen dabei beispielsweise Kneter oder verschiedene Mischertypen in Frage. Anschließend wird das oberflächenbeschichtete Material getrocknet und gegebenfalls gesiebt.

Der erfindungsgemäße Polyelektrolytzement kann übliche Beschleuniger oder Verzögerer aufweisen. Beispiele für Beschleuniger sind kurzkettige organische Säuren, wie z.B. Essigsäure, Alkohle, wie z.B. Ethylalkohol, Salze, wie z.B. Zinkacetat. Beispiele für Verzögerer sind organische Triole, wie z.B. Glycerin, einige organische Aminoalkohole, wie z.B. Triethanolamin (US-A-3,028,247).

Bei den Glasionomerzementen ist der Zusatz von Chelatbildnern zur Einstellung einer geeigneten Abbindung von besonderer Bedeutung (DE-A-23 19 715). Dafür kommen zahlreiche Verbindungen in Frage, vor allem solche, die Chelatbildungen ausbildende Hydroxy- oder Carboxylgruppen oder beide enthalten. Besonders hervorragende Ergebnisse wurden mit Weinsäure oder Citronensäure, insbesondere mit einem Gehalt von 5 Gew.-%, erzielt. Auch der Zusatz in Form eines Metallchelats zeigt den gewünschten Effekt.

im Sinne der vorliegenden Erfindung finden sich in den erfindungsgemäßen Polyelektrolytzementen 0 bis 10, vorzugsweise 0 bis 5 Gew.-% einer derartigen Verbindung, bevorzugt Weinsäure.

Außerdem kann die Abbindegeschwindigkeit auch durch die Behandlung der Oberfläche der Metallkationen-freisetzenden Verbindung eingestellt werden. Beispielsweise wird die Abbindegeschwindigkeit von Glasionomerzementen durch die Temperung des basischen Glases [Clinical Materials 12, 113 - 115 (1993)] oder die Dauer der Oberflächenbehandlung des basischen Glases mit Säure [DE-A-29 29 121] beeinflußt.
Neben dieser Abbindungsreaktion kann in dem erfindungsgemäßen Polyelektrolytzement bei Zusatz von polymerisierbaren Verbindungen und für die Polymerisation dieser Verbindungen geeigneten Initiatoren zusätzlich eine Polymerisationsreaktion ablaufen.

Unter einer polymerisierbaren Verbindung im obigen Sinne soll dabei eine Verbindung verstanden werden, die eine Polymerisationsreaktion eingehen kann. Derartige Verbindungen können beispielsweise zusätzliche Substitiuenten tragen, die mit der Metallkationen-freisetzenden Verbindung reagieren können, wie z.B. -COOH-Gruppen. Zur Verträglichkeit mit der durch das Abbindereaktionssystem, bestehend aus den Reaktionspartnern (a) und (b), hydrophilen Matrix ist es bevorzugt, wenn die polymerisierbaren Verbindungen hydrophilen Charakter haben. Beispiele für die verwendete polymerisierbare und/oder abbindbare Verbindung sind:

### (i) ethylenisch ungesättigte Verbindungen:

Vinyl-, Vinylether-, Acrylat-, Methacrylat-Verbindungen, die u.a. auch Hydroxygruppen enthalten können; Acrylate und Methacrylate, wie z.B. Methyl(meth)acrylat, n- oder i-Propyl(meth)acrylat, n-, i- oder tert.-Butyl(meth)acrylat und Hydroxyalkyl(meth)acrylat, Di(meth)acrylate des Propandiols, Butandiols, Hexandiols, Octandiols, Nonandiols, Decandiols und Eicosandiols, Di(meth)acrylate des Ethylenglycols, Diethylenglycols; Ester der (Meth)acrylsäure, wie beipielsweise Triethylenglycoldimethacrylat; Urethan(meth)acrylsäure; α-Cyanoacrylsäure; Crotonsäure; Zimtsäure; Sorbinsäure; (Meth)acrylamide wie z.B. Butylvinylether; Mono-N-Vinyl-Verbindungen wie N-Vinylpyrrolidon. Besonders bevorzugt sind Diacryl- und Dimethacrylsäureester des Bishydroxymethyltricyclo(5.2.1.0^{2,6})-decans; 2,2-Bis-4(3-methacryloxy-2-hydroxypropoxy)phenylpropan (bis-GMA); 3,6-Dioxaoctamethylendimethacrylat (TEDMA); 7,7,9-Trimethyl-4,13-dioxo-3,14,-dioxa-5,12-diazahexadecan-1,16-dioxy-dimethacrylat (UDMA);

### (ii) Epoxide der allgemeinen Formel

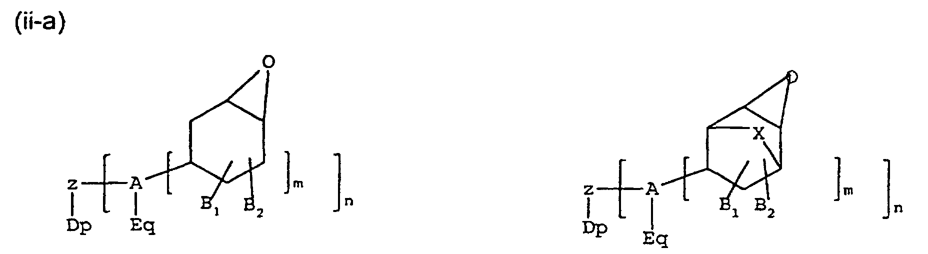

in welcher bedeuten:
- Z: einen aliphatischen, cycloaliphatischen oder aromatischen Rest mit 0 bis 22 C-Atomen oder eine Kombination dieser Reste, wobei ein oder mehrere C-Atome durch O, C=O, -O(C=)-, SiR₂ und/oder NR ersetzt sein können und wobei R ein aliphatischer Rest mit 1 bis 7 C-Atomen ist, wobei ein oder mehrere C-Atome durch O, C=O und/oder -(C=O)-- ersetzt sein können,
- A: einen aliphatischen, cycloaliphatischen oder aromatischen Rest mit 1 bis 18 C-Atomen oder eine Kombination dieser Reste, wobei ein oder mehrere C-Atome durch O, C=O, -O(C=)-, SiR₂ und/oder NR ersetzt sein können, wobei R ein aliphatischer Rest mit 1 bis 7 C-Atomen ist, bei dem ein oder mehrere C-Atome durch O, C=O und/oder -(C=O)- ersetzt sein können,
- B₁, B₂, D, E: unabhängig voneinander ein H-Atom oder einen aliphatischen Rest mit 1 bis 9 C-Atomen, wobei ein oder mehrere C-Atome durch O, C=O, -O(C=)-, SiR₂ und/oder NR ersetzt sein können und wobei R ein aliphatischer Rest mit 1 bis 7 C-Atomen ist, wobei ein oder mehrere C-Atome durch O, C=O und/oder -(C=O)- ersetzt sein können,
- n: 2 bis 7
- m: 1 bis 10
- p: 1 bis 5
- q: 1 bis 5 und
- x: CH₂, S oder O;

Diese Verbindungen und Möglichkeiten zu deren Herstellung sind in DE-A-196 48 283 oder WO 95/30402 beschriebenen.

Denkbar sind auch Epoxide der allgemeinen Formel 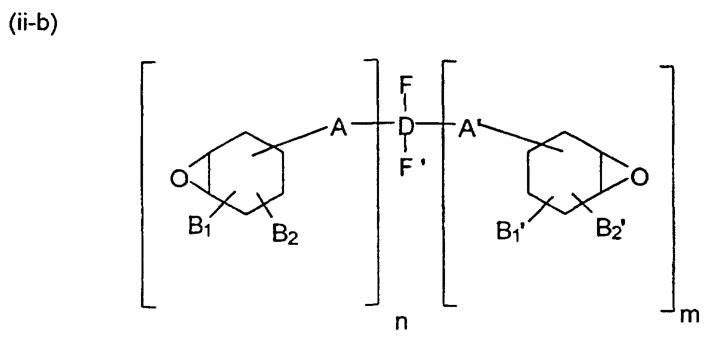 wobei
A, A' unabhängig voneinander einen unverzweigten oder verzweigten aliphatischen, cycloaliphatischen oder aromatischen Rest mit 0 bis 13 C-Atomen oder eine Kombination dieser Reste bedeuten, wobei ein oder mehrere C-Atome durch O, C=O, O(C=O), Si, N, S ersetzt sein können,
B1, B1', B2, B2' unabhängig voneinander H, einen unverzweigten oder verzweigten aliphatischen, cycloaliphatischen oder aromatischen Rest mit 0 bis 6 C-Atomen oder eine Kombination dieser Reste bedeuten, wobei ein oder mehrere C-Atome durch O, (C=O), O(C=O), Si, N, S ersetzt sein können,
F, F' unabhängig voneinander einen unverzweigten oder verzweigten aliphatischen, cycloaliphatischen oder aromatischen Rest mit 0 bis 10 C-Atomen oder eine Kombination dieser Reste bedeuten, wobei ein oder mehrere C-Atome durch O, (C=O), O(C=O), Si, N, S ersetzt sein können,
D einen unverzweigten oder verzweigten aliphatischen, cycloaliphatischen oder aromatischen Rest mit 1 bis 15 C-Atomen oder eine Kombination dieser Reste bedeutet, wobei mindestens ein C-Atom durch SiGG', SiG oder Si ersetzt ist und ein oder mehrere C-Atome durch O, (C=O), O(C=O), N oder S ersetzt sein können,
G, G' unabhängig voneinander H, einen unverzweigten oder verzweigten aliphatischen, cycloaliphatischen oder aromatischen Rest mit 0 bis 8 C-Atomen oder eine Kombination dieser Reste bedeuten, wobei ein oder mehrere C-Atome durch O, (C=O), O(C=O), Si, N, S ersetzt sein können,
n und m unabhängig voneinander 0, 1, 2 oder 3 bedeuten und n+m 2 bis 6 ergeben.
und wobei die Molmasse des Epoxids oder die mittlere Molmasse des Gemisches von Epoxiden 250 bis 1000 g/mol beträgt.

Diese Moleküle und die Verfahren zu ihrer Herstellung sind in der deutschen Patentanmeldung (Sipox) beschrieben.
(iii) durch Ringöffnungsmetathese polymerisierende Monomere oder Polymere mit folgender Struktur:

   M-Aₙ

   wobei
   - M: gleich H oder ein linearer, verzweigter, cyclischer oder polycyclischer organischer oder metallorganischer Rest ist. Organische Reste können C₁-C₃₀-Alkyl, C₆-C₂₀-Aryl, C₇-C₃₀-Alkaryl oder C₃-C₃₀-Cycloalkyl mit 0-10 Heteroatomen aus der Gruppe N, O, Si, P, S und einer Anzahl von n Anknüpfungspunkten für A sein. Metallorganische Reste enthalten neben den oben genannten organischen Resten zusätzlich lineare, verzweigte, cyclische oder polycyclische Gerüste anorganischer Natur.

Bevorzugte Reste M können 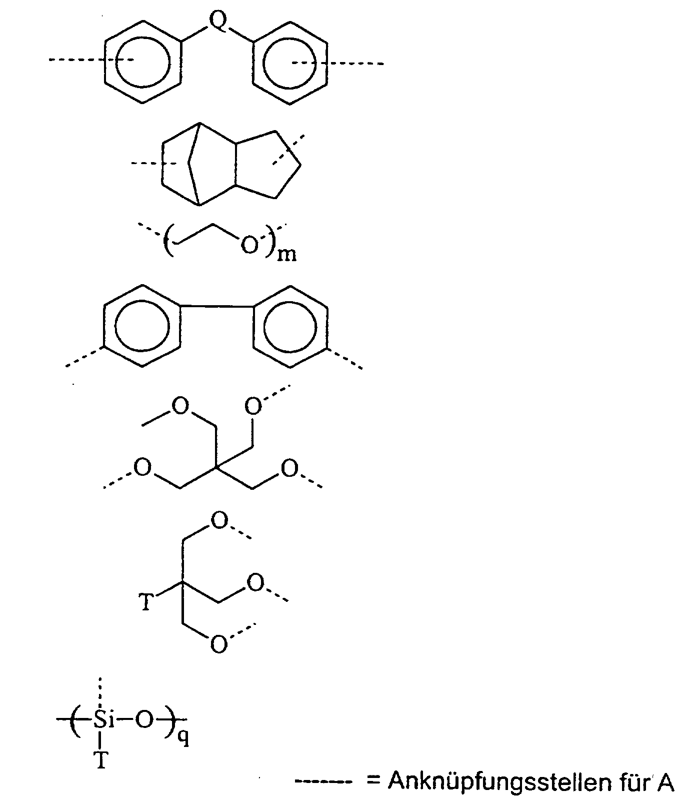 sein, mit der Maßgabe, daß Q gleich O, S, SO₂ oder ein linearer, verzweigter oder cyclischer C₁-C₂₀-Alkylenrest ist, der auch fluoriert sein kann, m eine ganze Zahl von 1-20 ist, T ein linearer, verzweigter oder cyclischer gesättigter oder ungesättigter C₁-C₂₀-Kohlenwasserstoffrest ist und q eine ganze Zahl von 3-20 ist.
- A: ist ein ungesättigter cyclischer oder polycyclischer organischer Rest der allgemeinen Formel

C-D,

wobei C gleich H oder ein linearer, verzweigter oder cyclischer gesättigter oder ungesättigter organischer C₁-C₂₀-Rest mit 0-10 Heteroatomen aus der Gruppe N, O, Si, P, S und 0-10 Carbonylgruppen ist und
- D: ein Cyclobutenyl-, Cyclopentenyl- oder ein an bezeichneter und wahlweise zusätzlich an einer weiteren Stelle im Ringsystem ungesättigter Rest der allgemeinen Formel 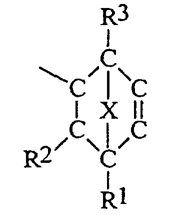 ist, in welcher bedeuten:
- R¹, R² , R³ H: oder einen linearen, verzweigten oder cyclischen gesättigten oder ungesättigten organischen C₁-C₂₀-Reste mit 0-10 Heteroatomen der Gruppe N, O, Si, P, S und 0-10 Carbonylgruppen, und
- X O, NH, S: bzw. einen gesättigten oder ungesättigten C₁-C₃₀-Kohlenwasserstoffrest.

Die Ringöffnungs-Metathese-Polymerisation wird auch in der Literatur beschrieben (Comprehensive Polymer Sci.; 4; S. 109-142).

Die polymerisierbaren Verbindungen können in den erfindungsgemäßen Polyelektrolytzementen zu 0 bis 30 Gew.-%, vorzugsweise zu 0 bis 20 Gew.-%, und in den Compomeren und kunststoffmodifizierten Glasionomerzementen beispielsweise zu 7 bis 15 Gew.-% enthalten sein.

Gegebenenfalls können als Initiatoren beispielsweise Katalysatoren für Heiß-, Kaltund/oder die Lichtpolymerisation zugesetzt werden. Hierbei können für die Heißpolymerisation beispielsweise Peroxide wie Dibenzoylperoxid, Dilaurylperoxid, tert.-Butylperoctoat oder tert.-Butylperbenzoat, aber auch α,α'-Azobis(isobutyroethylester), Benzpinakol und 2,2'-Dimethylbenzpinakol verwendet werden. Als Photoinitiatoren können alle Substanzen eingesetzt werden, die nach Bestrahlung mit UV- und/oder sichtbarem Licht die Polymerisation auslösen. Hierzu zählen z.B. Diazoniumverbindungen (US-A-3,205,157), Sulfoniumverbindungen (US-A-4,173,476), lodoniumverbindungen (US-A-4,264,703, US-A-4,394,403) oder Bisacylphosphinoxide (EP-A-184095). Weitere Photoinitiatoren sind z.B. α-Diketone wie bevorzugt 9,10-Phenantrenchinon, Diacetyl, Furil, Anisil, 4,4'-Dichlorbenzil, 4,4'-Dialkoxybenzil und Campherchinon. Initiatoren zur kationischen Lichthärtung sind z.B. die in der DE-A-197 36 471 beschriebenen Verbindungen. Weitere kationische Polymerisationsinitiatoren sind in der DE-A-25 15 593 und der WO 96/13538 beschrieben. Für die Kaltpolymerisation eignen sich vor allem die bekannten, Radikale liefernden Initiatorsysteme auf Peroxid/Amin- bzw. Peroxid / Sulfitur- und / oder Barbitursäure-Basis, wie z.B. Benzoyl- bzw. Lauroylperoxid mit NN-Dimethylsym. Xylidin und NN-Dimethyl-p-toluidin.

Katalysatoren, die für die Polymerisation nach Ringöffnungs-Metathese zugesetzt werden können, sind beispielsweise Radikal- oder Kationenbildner und die in der WO 96/23829 oder die von van der Schaaf, Hafner, Mühlebach in "Angewandte Chemie", 1996, 108, S. 1974-1977 beschriebenen Verbindungen.

Übliche Beschleuniger für die Polymerisationsreaktion, die zugesezt werden können, sind beispielsweise oxidierend wirkende Zusatzstoffe wie Hydroperoxide (z.B. Cumolhydroperoxid, Dialkylperoxide), Perester (z.B. tert.-Butylperbenzoat, tert.-Butylisononanoat) oder anorganische Oxidationsmittel (z.B. Kaliumpersulfat, Natriumperborat) oder andere radikalerzeugende Zusatzstoffe, wie Diaryliodoniumverbindungen, aromatische Amine, Alkylamine oder aromatische Alkylamine.

Die Initiatoren, Katalysatoren und im vorherstehenden Absatz beschriebenen Beschleuniger etc. können in den erfindungsgemäßen Polyelektrolytzementen zu 0 - 1 Gew.-% enthalten sein.

Ferner kann der erfindungsgemäße Polyelektrolytzement Hilfsstoffe, wie Farbstoffe, Pigmente, Röntgenkontrastmittel, Fließverbesserer, Thixotropiemittel, polymere Verdickungsmittel oder Stabilisatoren, aufweisen. Übliche Füllstoffe für Dentalwerkstoffe sind beispielsweise Glas- und Quarzpulver, Kunststoffpulver, pyrogene hochdisperse Kieselsäuren sowie Mischungen dieser Komponenten. Diese sonstigen Zusätze sind in den erfindungsgemäßen Polyelektrolytzementen zu 0 - 60 Gew.-% enthalten.

Die genannten Füllstoffe können auch durch z.B. eine Behandlung mit Organosilanen bzw. -siloxanen oder durch die Veretherung von Hydroxylgruppen zu Alkoxygruppen hydrophobiert sein.

In einer besonderen Ausgestaltung kann zumindest ein Teil der in fester Form vorliegenden Bestandteile des erfindungsgemäßen Polyelektrolytzements in pulvriger, granulierter und / oder tablettierter Form dargereicht werden.

Unter Granulaten werden sedimentierte Anhäufungen von Granulatkörnern verstanden. Ein Granulatkorn ist ein zusammengekittetes, asymmetrisches, keine harmonische geometrische Form aufweisendes Aggregat oder Agglomerat aus Pulver- bzw. Staubteilchen, die meist bessere Fließeigenschaften als Pulvermischungen haben [Ullmanns Encyklopädie der technischen Chemie, Verlag Chemie, Weinheim, 4. Auflage, 1979, 18-157ff]. Die Oberfläche des Korns, das kugel-, stäbchen- oder zylinderförmig sein kann, ist uneben und gratförmig [H. P. Fiedler, Lexikon der Hilfsstoffe für Pharmazie, Kosmetik und angrenzende Gebiete, Editio Cantor Verlag Aulendorf, 4. Aufl., 1996, S. 722].

Die Granulierung bietet im praktischen Handling von Pulvern viele Vorteile gegenüber dem feindispersen Zustand staubförmiger Produkte. Insbesondere die Belästigung der Umwelt durch Staubemissionen, ein definiertes Fließverhalten, ein einfacheres Handling bei Produktion und Abfüllung und auch ein schnelleres Dispergieren bzw. Lösen verbessern die Handhabung derartiger Produkte erheblich [Rumpf, Chemie-Ingenieur-Technik 30 bzw. 46].

Damit feindisperse Pulver zu Granulaten geformt werden können, sind Haftkräfte zwischen den einzelnen Körnern notwendig. Die Haftkräfte können beispielsweise durch Festkörperbrücken, wie Sinterung, chemische Reaktion, erhärtende Bindemittel oder durch Kristallisation zwischen den einzelnen Partikeln ausgebildet werden. Weitere Bindemöglichkeiten sind Grenzflächenkräfte an freibeweglichen Flüssigkeitsoberflächen, Adhäsions- und Kohäsionskräfte, Anziehungskräfte zwischen Feststoffteilchen oder formschlüssige Bindungen.

In der Pharmaindustrie ist die sogenannte "Klebstoffgranulierung" weit verbreitet. Die erhaltenen Granulate werden entweder als solche eingesetzt oder in der Tablettierung weiterverarbeitet. Bei der Klebstoffgranulierung kommen als Bindemittel (Klebstoffe, Granulierhilfsmittel) ähnliche Verbindungen zum Einsatz, wie auch bei der Herstellung von Filmüberzügen. Neben den in der Tabelle genannten Verbindungen können z.B. noch eingesetzt werden: Gelatine, Stärke, Alginate. [F. Gstimer, Grundstoffe und Verfahren der Arzneibereitung, Ferdinand Enke Verlag, Stuttgart, 1960, S. 25 ff; P.H. List et. al., Arzneiformenlehre, 4. Aufl., Wissenschaftliche Verlagsgesellschaft mbH, Stuttgart, 1985, S. 84 ff]

Werden die erfindungsgemäßen Polyelektrolytzemente in Form von Pulver-Flüssigkeitssystemen dargereicht, so ist das Anmischen oftmals sehr schwierig, weil die Pulver sehr große Oberflächen besitzen (s.o.), die ein Anmischen sehr mühsam machen. Außerdem stehen sich bei einem für gute mechanische Werte oftmals erforderlichem hohen Pulver-Flüssigkeitsverhältnis eine große Menge Pulver und eine nur geringe Menge Flüssigkeit gegenüber. Durch das gegenüber dem Partikelvolumen relativ geringe Flüssigkeitsvolumen und der hohen spezifischen Oberfläche des Zementes, die es zu benetzen gilt, ist der Anmischvorgang erschwert.

Durch eine Granulierung kann die Benetzung des Pulvers mit der Flüssigkeit beschleunigt werden, weil bei der Granulierung das Pulver innig miteinander verbunden wird, wodurch poröse Strukturen entstehen. Durch diese Struktur wird die Flüssigkeit aufgrund von Kapillarkräften beschleunigt in das Granulat aufgenommen, was die Anmischzeit erheblich verkürzt.

Die Anwendung von Granulaten für zahnärztliche Applikationen wurde bereits in JP-A-06321724 , JP-A-53023190, DE-A-35 11 721 dargestellt. In der DE-A-35 11 721, die die Granulierung von Alginat-Abfomrmassen beschreibt, werden Granulierhilfsmittel beschrieben, ohne die eine Granulierung nicht möglich ist. Außerdem handelt es sich bei den granulierten Materialien nicht um gemäß der vorliegenden Erfindung zumindest partiell oberflächlich beschichtete Materialien.

Granulierhilfsmitteln, die üblicherweise zur Herstellung des Partikelverbundes verwendet werden, sind in den erfindungsgemäßen Systemen nicht einsetzbar, da die Menge, die hierfür erforderlich ist, die physikalischen Eigenschaften des ausgehärteten Polyelektrolytzements, insbesondere die Abbindereaktion, negativ beeinflussen würde (s.o.).

Beispielsweise wird Eudragit® E aufgrund seiner Klebkraft in der Pharmaindustrie als gängiges Granulierhilfsmittel eingesetzt. Die Eudragit® -Konzentrationen bewegen sich dabei in einem Bereich von 5 - 10 Gew.-%, bezogen auf das Granulat. Geringere Konzentrationen an Eudragit® führen aufgrund der beschränkten Klebekraft nicht zu den gewünschten Granulaten, d.h. der Feinanteil wäre zu groß, um den gewünschten Effekt zu erzielen.

Erfindungsgemäß wird ein Granulat aus mindestens einem Teil der in fester Form vorliegenden Rezepturbestandteile des Polyelektrolytzements zur Verfügung gestellt. Dabei dient - im Sinne einer autogenen Granulierung - zumindest ein Teil des Reaktionspartners (b) als wesentliches Granuliermittel, und das Granulat-zerfällt im Kontakt mit den flüssigen Rezepturbestandteilen wieder in das Primärkom, so daß die flüssigen und festen Bestandteile des Polyelektrolytzements durch den Eintrag mechanischer Energie leicht innig miteinander vermischt werden können.

Der Eintrag der mechanischen Engergie kann dabei unterschiedlich erfolgen. Bei Handmischvarianten erfolgt der Energieeintrag beispielsweise durch Anspateln, bei Einmalapplikationskapseln durch Schütteln der Kapsel in einem automatischen Mischgerät.

Vorteile der erfindungsgemäßen Ausgestaltung sind u.a. eine reduzierte Anmischzeit der erfindungsgemäßen Polyelektrolytzemente. Dies führt zu einer Verkürzung der Anmischzeit von in Einmalapplikationskapseln verpackten Polyelektrolytzementen in auf dem Markt befindlichen automatischen Mischsystemen und zu einer deutlichen Erleichterung des Anmischens bei den sog. 'Handmischvarianten' des erfindungsgemäßen Polyelektrolyzementes in granulärer Darreichungsform.

Unter "autogener Granulierung" soll im Sinne der vorliegenden Erfindung verstanden werden, daß die Granulierung im wesentlichen ohne den zusätzlichen Einsatz von Graunlierhilfsmitteln erfolgt. Es dient vielmehr der zumindest teilweise in der Pulvermischung befindliche Polyelektrolyt als Granulierhilfsmittel.

Überraschenderweise lassen sich damit zwei eigentlich miteinander reaktive Substanzen granulieren, ohne daß eine Reaktionen zwischen ihnen ausgelöst wird. Das erfindungsgemäße Granulat zeichnet sich somit dadurch aus, daß bei der Herstellung keine nennenswerten Mengen Granulierhilfsmittel eingesetzt werden und somit das Granulat keine nennenswerten Mengen üblicher Granuliermittel enthält.

Zur Auslösung der Abbindereaktion wird das Granulat mit der Flüssigkeit gemischt. Aufgrund der sehr schnellen Wasseraufnahme durch die Kapillaren des Granulates wird das Granulat bis auf das Primärkorn wieder zerstört, so daß geringe Filmstärken gewährleistet sind. Die Flüssigkeit wird dabei aufgesaugt und es resultiert eine leichte Anmischbarkeit.

Das erfindungsgemäße Granulat weist für die dentale Anwendung bevorzugt eine mittlere Korngröße zwischen 10 und 1000 µm, besonders bevorzugt zwischen 50 und 500 µm, auf.

Im Stand der Technik sind mehrere bekannte Verfahren zur Herstellung von Granulaten beschrieben (s. beispielsweise [Ullmanns Encyklopädie der technischen Chemie, Verlag Chemie, Weinheim, 4. Auflage, 1979, 18-157ff]). Gängige Verfahren sind dabei die Feucht-, die Schmelz- und die Trockengranulierung. Diese Verfahren sind prinzipiell zur Herstellung des erfindungsgemäßen Granulates geeignet.

Besonders bevorzugt ist die Durchführung einer Feuchtgranulierung mit einem Lösungsmittel oder einem Lösungsmittelgemisch, das zumindest einen Teil des Reaktionspartners (b) anlöst, ohne eine Reaktion zwischen den beiden (a) und (b) auszulösen, und nach der Granulierung entfernt wird. Die Feuchtgranulierug erzeugt besonders hohe Porositäten, wodurch sich die Anmischzeiten gut reduzieren lassen. Durch den Zusatz von Lösungsmitteln bei der Granulierung wird die Polysäure angelöst und dadurch kommt der Verbund zum Granulat zustande. Die Auswahl des Lösungsmittels muß dabei so gestaltet werden, daß die Polysäure angelöst, allerdings keine Reaktion ausgelöst wird. Besonders bevorzugt weisen die zur Feuchtgranulierung eingesetzten Lösungsmittel polare Eigenschaften auf. Ganz besonders bevorzugt handelt es sich bei dem eingesetzten Lösungsmittelgemisch vorwiegend um kurzkettige Alkohole. Die Granuliermischung wird in Mischern durch die Zugabe von Lösungsmitteln hergestellt. Das Lösungsmittel wird nach der Granulierung wieder verdampft.

Bei der Trockengranulierung handelt es sich um ein weiteres bevorzugtes Verfahren zur Herstellung des erfindungsgemäßen Granulates. Dabei werden die zu granulierenden Stoffe durch den Eintrag von mechanischer Energie zu Schülpen verpreßt. Diese Preßlinge werden in einem nachfolgenden Zerkleinerungsschritt in Mühlen zerstört. Das dabei entstehende Bruchgranulat wird klassiert. Die Granulate, die eine Korngröße außerhalb der gewünschten Grenzen haben, werden erneut zu Schülpen verpreßt. Die Granulate mit dem geforderten Korngrößenspektrum werden der Abfüllung zugeführt.

Im Rahmen der vorliegenden Erfindung hat sich auch herausgestellt, daß sich alleine durch die Granulierung ohne vorherige Oberflächenbeschichtung eine Verbesserung der Lagerstabilität erreichen läßt.

Betroffen sind davon ein- oder mehrkomponentige Polyelektrolytzemente, enthaltend mindestens zwei Reaktionspartner, (a) mindestens eine Metallkationen-freisetzende Verbindung und (b) einen oder mehrere in den Festzustand überführbare Polyelektrolyte, wobei mindestens einer der Polyelektrolyte wenigstens teilweise wasserlöslich ist, wobei - im Sinne einer autogenen Granulierung - zumindest ein Teil des Reaktionspartners (b) als wesentliches Granuliermittel zur Granulierung von mindestens einem Teil der in fester Form vorliegenden Rezepturbestandteile des Polyelektrolytzements dient.

Die erfindungsgemäßen Polyelektrolytzemente und Granulate können zur Herstellung von Dentalmaterialien verwendet werden, insbesondere zur Herstellung von Füllungsmaterialien, Befestigungsmaterialien, Unterfüllungsmaterialien, Stumpfaufbau- und Ergänzungsmaterialien [A. D. Wilson, J. W. McLean, Glasionomerzement, Quintessenz Verlags-GmbH, Berlin 1998], endodontischen Materialien, insbesondere für die ortho- und retrograde Füllung [EP-C-04 69 573], Fissurenversiegelungsmaterialien [J. W. McLean, A. D. Wilson, Br. Dent. J. 136, 269-276, 1974], Befestigungsmaterialien für die Kieferorthopädie [H. W. Seehozer, Schweiz. Monatsschr. Zahnmed. 97, 344-347,1987] oder von Füllungsmaterialien für offene Furkationen [C. Hüskens, C. Matter-Grütter, F. Lutz, Schweiz. Monatsschr. Zahnmed. 105, 216-221, 1995].

Die erfindungsgemäßen Polyelektrolytzemente und/oder Granulate werden üblicherweise in Behältnisse, wie Mischkapseln, Kartuschen, Tuben und Gläser abgepackt. Je nach Behältnis, das zur Verpackung eingesetzt wird, werden unterschiedliche Geräte zum Anmischen und/oder zur Applizierung verwendet.

### BEISPIELE

### Oberflächenbeschichtung

Die nachfolgenden Ausführungsbeispiele beschreiben verschiedene erfindungsgemäße Polyelektrolytzemente. Analog zu den erfindungsgemäßen Beispielen 2 - 10 wurde das Beispiel 1 (Vergleich) und analog zu dem erfindungsgemäßen Beispiel 12 wurde das Beispiel 11 (Vergleich) ohne Zusatz eines organischen Oberflächenbeschichtungsmittels hergestellt. Beispiel 12a dient ebenfalls zum Vergleich.

In allen Beispielen wurden Metallkationen-freisetzende Verbindungen und Flüssigkeiten von handelsüblichen Produkten eingesetzt (ESPE Dental AG). Ketac-Molar® -Pulver enthält ein Glas für Glasionomerzemente und Durelon® -Pulver ein ZnO für Polycarboxylatzemente.

Für die Beschichtung der Metallkationen-freisetzenden Verbindung wurden die Beschichtungsmittel in dem in der Tabelle angegebenen Lösungsmittel vorgelöst. Die Konzentration des Lösungsmittels wurde jeweils so eingestellt, daß das Pulver optimal vom Lösungsmittel benetzt wurde. Nach Durchmischen des Pulvers in einem Labormischer wurde dieses etwa 3 h bei ca. 100°C getrocknet.

Die Pulver wurden ohne bzw. nach Beschichtung mit trockner Säure am Röhnrad ca. 30 min gemischt. Eingesetzt wurde bei den Versuchen mit Glasionomerzementen ein Copolymer aus Acryl- und Maleinsäure (Ketac-Molar® -Flüssigkeit) bzw. reine Polyacrylsäure (Durelon® -Flüssigkeit) bei den Versuchen mit Polycarboxylatzement. Art und Konzentration des Beschichtungsmittels (bezogen auf die Metallkationen-freisetzende Verbindung) sowie die Konzentration der Polyalkensäure in der Endmischung sind in Tabelle 1 wiedergegeben.

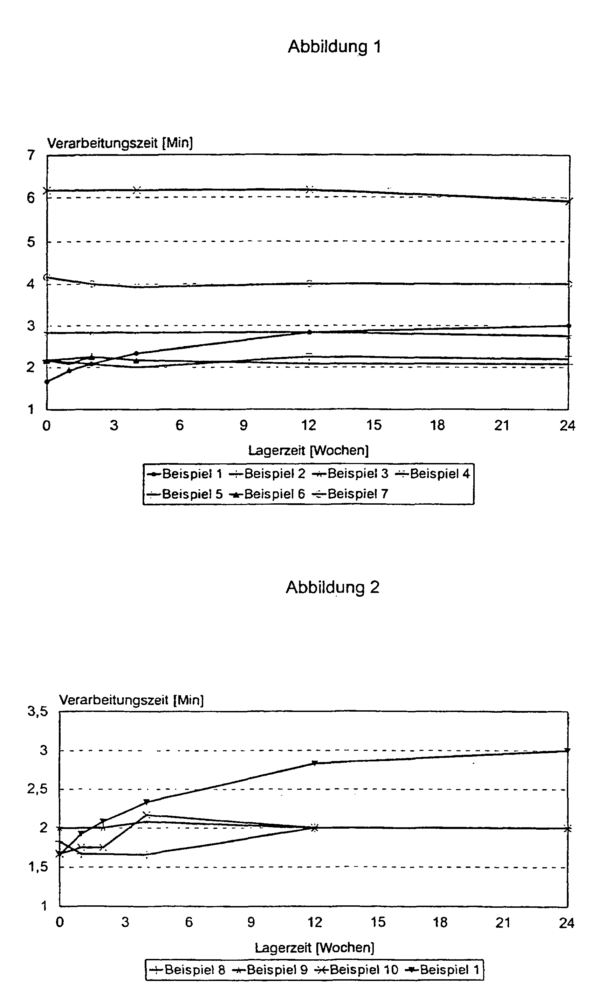

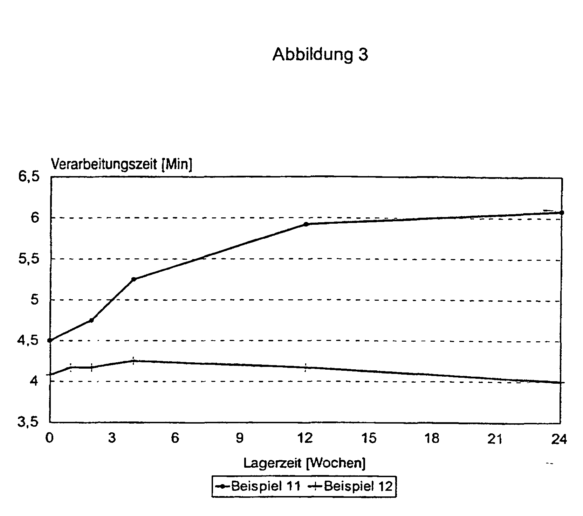

Zur Messung der Abbindung wurden die beiden in fester Form vorliegenden und homogen vermischten Reaktionspartner, Metallkationen-freisetzende Verbindung und der Polyelektrolyt, im angegebenen Pulver-Flüssigkeitsverhältnis (P/F) mit der der Tabelle 1 zu entnehmenden Flüssigkeit gemischt, und die Abbindungen wurden an einem Rheometer Typ Curometer (Shawberry) bestimmt. Die in der Tabelle angegebenen Werte beziehen sich auf den Abbindebeginn (tₐ). Die Tabelle 2 und die Abbildungen 1 - 3 geben den Verlauf der Abbindung bei Lagerung unter 50% relative Feuchte bei 23°C wieder.

Die Herstellung von Eudragit® E-Tartrat erfolgte durch Neutralisation von 45 g Eudragit® E mit 11 g Weinsäure in wäßriger Lösung.

### Beispiel 12a (Vergleich)

Eine Mischung bestehend aus 95% Ketac-Molar Glaspulver und 5% Eudragit E-Tartrat werden mit Ketac-Molar-Flüssigkeit zur Abbindung gebracht. Aufgrund der starken Verdickung der Zementmischung kann nur ein Pulver-Flüssigkeitsverhältnis von 2,4 eingestellt werden. Die in der Tabelle 3 wiedergegebenen mechanischen Eigenschaften zeigen einen deutlichen Abfall sowohl gegenüber dem Vergleichszement, als auch gegenüber einem Zement der im erfindungsgemäßen Konzentrationsbereich beschichtet wurde:

**Tablelle 1:**

| Versuchsbedingungen. | | | | | | | | |
|---|---|---|---|---|---|---|---|---|
| Beispiel Nr. | Metallkationen freisetzende Verbindung | Beschichtungsmittel | | Lösungsmittel | Polyelektrolyt in Pulvermischung | | Flüssigkeit | P/F [g/g] |
| | | Art | Konz. [%] | | Art | Konz. [%] | | |
| 1 (Vergleich) | Ketac Molar® | - | 0 | - | Copolymer | 5 | Ketac Molar® | 3,4 |
| 2 | Ketac Molar® | Eudragit® E | 0,5 | 1-Propanol | Copolymer | 5 | Ketac Molar® | 3,4 |
| 3 | Ketac Molar® | Eudragit® E | 0,5 | Aceton | Copolymer | 5 | Ketac Molar® | 3,4 |
| 4 | Ketac Molar® | Eudragit® E | 0,2 | Aceton | Copolymer | 5 | Ketac Molar® | 3,4 |
| 5 | Ketac Molar® | Eudragit ® Etartrat | 1,24 | Wasser | Copolymer | 5 | Ketac Molar® | 3,4 |
| 6 | Ketac Molar® | Eudragit® Etartrat | 0,25 | Wasser | Copolymer | 5 | Ketac Molar® | 3,4 |
| 7 | Ketac Molar® | Eudragit® Etartrat | 0,62 | Wasser | Copolymer | 5 | Ketac Molar® | 3,4 |
| 8 | Ketac Molar® | Copolymer 845 - ISP | 0,5 | Wasser | Copolymer | 5 | Ketac Molar® | 3,2 |
| 9 | Ketac Molar® | OPADRY® 11 - Colorcon | 0,5 | Wasser | Copolymer | 5 | Ketac Molar® | 3,4 |
| 10 | Ketac Motar® | Surelease - Colorcon | 0,5 | Wasser | Copolymer | 5 | Ketac Molar® | 3,4 |
| 11 (Vergleich) | Durelon® | - | 0 | - | Polyacrylsäure | 18 | Durelon® | 3,2 |
| 12 | Durelon® | Eudragit® E | 0,5 | 1-Propanol | Polyacrylsäure | 18 | Durelon® | 3,2 |

**Tabelle 2:**

| Versuchsergebnisse (Abbindebeginn tₐ in Min:Sec). | | | | | | |
|---|---|---|---|---|---|---|
| **Beispiel Nr.** | **Startwert** | **Lagerzeit [Wochen]** | | **Lagerzeit [Monate]** | | |
| | | **1** | **2** | **1** | **3** | **6** |
| 1 (Vergleich) | 1:40 | 1:55 | 2:05 | 2:20 | 2:50 | 3:00 |
| 2 | 2:10 | 2:05 | 2:15 | 2:10 | 2:05 | 2:05 |
| 3 | 2:50 | | 2:50 | 2:50 | 2:50 | 2:75 |
| 4 | 2:10 | | 2:05 | 2:00 | 2:15 | 2:12 |
| 5 | 6:10 | | | 6:10 | 6:10 | 5:55 |
| 6 | 2:10 | | 2:15 | 2:10 | | |
| 7 | 4.10 | | 4:00 | 3:55 | 4:00 | 4:00 |
| 8 | 1:50 | 1:40 | | 1:40 | 2:00 | 2:00 |
| 9 | 2:00 | | 2:00 | 2:05 | 2:00 | 2:00 |
| 10 | 1:40 | 1:45 | 1:45 | 2:10 | 2:00 | 2:00 |
| 11 (Vergleich) | 4:30 | | 4:45 | 5:15 | 5:55 | 6:05 |
| 12 | 4:05 | 4:10 | 4:10 | 4:15 | 4:10 | 4:00 |

Die Ergebnisse dieser Versuche zeigen, daß die erfindungsgemäßen Polyelektrolytzemente der Beispiele 2 - 10 und 12 im Rahmen der Meßgenauigkeit der Methode (+/- 15 sec) eine ausgezeichnete Lagerstabilität aufweisen, während die beiden Vergleichsbeispiele bereits nach 4 Wochen eine deutliche Zunahme der Abbindezeit zeigen, die sich bei Lagerung weiter fortsetzt.

Exemplarisch ist außerdem der folgenden Tabelle 3 zu entnehmen, daß beispielsweise bei dem erfindungsgemäßen Beispiel 6 im Vergleich zu Beispiel 1 (Vergleichsbeispiel) die mechanischen Werte durch das Oberflächenbeschichtungsmittel nicht negativ beeinflußt werden. Die zur Messung der Druck- und Biegefestigkeit erforderlichen Prüfkörper wurden gemäß der Norm ISO 9917 bzw. nach ESPE-eigener Norm in Anlehnung an die Norm ISO 4049 in MPa bestimmt. Abweichend von ISO 4049 (Prüfkörper mit den Maßen 15*2*2 mm) wurde ein Prüfkörper mit den Maßen 12*2*2 mm verwendet.

**Tabelle 3:**

| Druck- und Biegefestigkeiten des erfindungsgemäßen Beispiels 6 im Vergleich zu Beispiel 1 (Standardabweichungen in Klammern). | | |
|---|---|---|
| | Druckfestigkeit [MPa] | Biegefestigkeit [MPa] |
| Beispiel 1 (Vergleich) | 224 (20) | 53 (12) |
| Beispiel 6 | 248 (21) | 56 (9) |
| Beispiel 12a (Vergleich) | 147 (17) | 25 (7) |

Die Ergebnisse dieser Versuche zeigen, daß mit den erfindungsgemäßen Polyelektrolytzementen eine verbesserte Lagerstabilität erreicht wird, ohne daß durch das Oberflächenbeschichtungsmittel die mechanischen Werte im Vergleich zu einem nicht oberflächlich beschichteten Vergleichsmaterial negativ beeinflußt werden.

### Granulierung

### Beispiel 13

Es wurde eine autogen granulierte Pulvermischung aus 18 Teilen Polysäure und 100 Teilen Glaspulver, oberflächlich beschichtet analog Beispiel 7, hergestellt, indem 12 Teile Isopropanol zugesetzt und in einem Pharmamischer wenige Minuten homogenisiert wurden. Um den gewünschten Korngrößenbereich einzustellen, wurde die feuchte Mischung anschließend über ein Sieb einer Maschenweite von 300 bis 500 µm gesiebt. Das Lösungsmittel wurde durch eine anschließende ca. zweistündige Trocknung bei 100°C entfernt.

### Beispiel 14 (Vergleich)

Pulvermischung analog erfindungsgemäßem Beispiel 13, jedoch wurden weder die Oberflächenbeschichtung noch die Granulierung durchgeführt.

Zur Ausprüfung der physikalischen Eigenschaften der nach Beispiel 13 und 14 erhältlichen Polyelektrolytzemente nach Norm ISO 9917 werden Pulver und Flüssigkeit (Wasser mit 17 Gew.-% Weinsäure) im Verhältnis 3,8 gemischt. In Tabelle 4 sind ausgewählte physikalische Eigenschaften des Polyelektrolytzements nach dem erfindungsgemäßen Beispiel 13 den entsprechenden Eigenschaften der Polyelektrolytzemente nach Beispiel 14 (Vergleich) und dem noch folgenden Beispiel 15 (Vergleich) gegenübergestellt.

**Tabelle 4:**

| Physikalische Eigenschaften des aus dem Granulat des erfindungsgemäßen Beispiels 13 erhaltenen Polyelektrolytzements im Vergleich zu aus Beispiel 14 (Vergleich) und 15 (Vergleich) erhaltenen Polyelektroylytzementen. | | | |
|---|---|---|---|
| | Beispiel 13 | Beispiel 14 (Vergleich) | Beispiel 15 (Vergleich) |
| Druckfestigkeit [MPa] | 127 | 145 | 45 |
| Oberflächenhärte [MPa] | 81 | 51 | 22 |
| Filmstärke [µm] | 13 | 10 | 134 |

Die nicht granulierte Mischung (Beispiel 14 (Vergleich)) zeigt bei der Lagerung in der Glasflasche Entmischungserscheinungen, so daß das Produkt vor der Verwendung aufgeschüttelt werden muß. Dabei tritt eine starke Staubentwicklung auf, so daß bei der Entnahme des Pulvers die Arbeitsfläche häufig mit Pulver verschmutzt wird. Das Anmischen des Pulvers mit der Flüssigkeit ist nur durch einen erhöhten Eintrag von mechanischer Energie möglich.

Das Granulat (erfindungsgemäßes Beispiel 13) zeigt dagegen keine Entmischungserscheinungen in der Glasflasche. Das Granulat staubt nicht und läßt sich gut entnehmen und sehr gut dosieren. Es wurde beim Anmischen mit Wasser sehr schnell benetzt und ergibt einen sehr dünnen Film. Der granulierte Polyelektrolytzement erfüllt alle Anforderungen.

Darüber hinaus wurden die Anmischzeiten von aus Beispiel 13 und 14 (Vergleich) erhältlichen Pulvern mit Flüssigkeit (Wasser mit 17 Gew.-% Weinsäure) untersucht. Dazu wurden je 380 mg Pulver und 100 mg Flüssigkeit auf einem Anmischblock abgewogen. Die Zeitmessung startete, als der Flüssigkeitstropfen das Pulver berührte und endete beim Vorliegen eines homogenen Zementes ohne Pulvernester. Die ermittelten Daten sind in Tabelle 5 wiedergegeben.

**Tabelle 5:**

| Mischzeiten. | | |
|---|---|---|
| Anmischversuch Nummer | Beispiel 13 Anmischzeit [sec] | Beispiel 14 Anmischzeit (Vergleich) [sec] |
| 1 | 12 | 32 |
| 2 | 11 | 35 |
| 3 | 13 | 34 |
| 4 | 10 | 28 |
| 5 | 12 | 38 |
| 6 | 10 | 32 |
| 7 | 11 | 34 |
| 8 | 14 | 29 |
| 9 | 10 | 29 |
| 10 | 10 | 34 |
| Mittelwert (Standardabweichung) | 11,3 (1,3) | 32,5 (3,0) |

Das Ergebnis dieser Versuche zeigt, daß die erfindungsgemäßen Granulate deutlich reduzierte Anmischzeiten bis zum Erhalt eines homogen gemischten Polyelektrolytzements zeigen (Zeitreduktion um über 60%). Dies ist auf die erheblich verbesserte Benetzung der Granulate mit Flüssigkeit im Vergleich zum Beispiel 14 (Vergleich) zurückzuführen.

### Beispiel 15 (Vergleich)

Die Pulvermischung aus Beispiel 14 (Vergleich) wurde in einem Pharmamischer vorgelegt und mit 5 % Eudragit® E bezogen auf die granulierte Pulvermischung granuliert. Anschließend wurde über ein Sieb einer Maschenweite von 300 bis 500 µm gesiebt, um den gewünschten Korngrößenbereich analog dem erfindungsgemäßen Beispiel 13 einzustellen. Durch eine Trocknung bei 100°C wurde das Lösungsmittel wieder entfernt. Das granulierte Pulver wurde wie bei Beispiel 13 und 14 beschrieben angemischt und der resultierende Polyelektrolytzement ausgeprüft. Die ermittelten Daten sind Tabelle 4 zu entnehmen.

Der Vergleich mit nicht oberflächlich behandeltem und nicht granuliertem Pulver aus Beispiel 14 (Vergleich) zeigt, daß bereits die zur Granulierung empfohlene Mindestmenge von ca. 5% Granulierhilfsmittel (s.o.) bezogen auf die granulierte Pulvermischung die physikalischen Eigenschaften des Polyelektrolytzements stark verschlechtert. Dies zeigt, daß der Einsatz von gängigen Granuliermitteln zum Erhalt erfindungsgemäßer Granulate nicht geeignet ist.

## Patentansprüche

1. Ein- oder mehrkomponentiger Polyelektrolytzement enthaltend mindestens zwei Reaktionspartner:
(a) mindestens eine Metallkationen-freisetzende Verbindung, und
(b) einen oder mehrere in den Festzustand überführbare Polyelektrolyte, wobei mindestens einer der Polyelektrolyte wenigstens teilweise wasserlöslich ist,
wobei mindestens ein Teil der Reaktionspartner (a) und / oder (b) mit einem organischen Oberflächenbeschichtungsmittel zumindest teilweise überzogen ist, wobei das organische Oberftächenbeschichtungsmittel ein filmbildendes Material ist und in einer Menge von 0,01 bis 3 Gew.-%, bezogen auf das Gesamtgewicht des oberflächenbeschichteten Materials, vorliegt.

2. Polyelektrolytzement nach Anspruch 1, wobei sich die beiden Reaktionspartner (a) und (b) zumindest teilweise in derselben Komponente des Polyelektrolytzementes befinden.

3. Polyelektrolytzement nach einem der vorhergehenden Ansprüche,
wobei mindestens ein Teil der in derselben Komponente befindlichen Reaktionspartner (a) und / oder (b) mit einem organischen Oberflächenbeschichtungsmittel überzogen ist.

4. Polyelektrolytzement nach einem der vorhergehenden Ansprüche,
wobei das organischen Oberflächenbeschichtungsmittel zumindest teilweise säurelöslich ist.

5. Polyelektrolytzement nach einem der vorhergehenden Ansprüche,
wobei zumindest ein Teil der in fester Form vorliegenden Bestandteile in pulvriger, granulierter und / oder tablettierter Form vorliegt.

6. Verfahren zur Herstellung eines Polyelektrolytzements nach einem der vorhergehenden Ansprüche in Granulatform, wobei zumindest ein Teil eines oder mehrerer in den Festzustand überführbaren Polyelektrolyte, die wenigstens teilweise wasserlöslich sind, als wesentliches Granuliermittel dient und das Granuliermittel die Oberfläche eines zu granulierenden Bestandteils zumindest teilweise bedeckt.

7. Verfahren nach Anspruch 6, wobei das Verfahren als Feuchtgranulierung mit einem Lösungsmittel oder einem Lösungsmittelgemisch durchgeführt wird, das zumindest einen Teil des Reaktionspartners (b) anlöst, ohne eine Reaktion zwischen (a) und (b) auszulösen, und das nach der Granulierung entfernt wird.

8. Verfahren nach einem der Ansprüche 6 bis 7, wobei die zur Feuchtgranulierung eingesetzten Lösungsmittel polare Eigenschaften aufweisen.

9. Verfahren nach Anspruch 6, wobei eine Trockengranulierung durchgeführt wird.

10. Granulat erhältlich durch ein Verfahren gemäß den Ansprüchen 6 bis 9.

11. Granulat nach Anspruch 10, das eine mittlere Komgröße zwischen 10 und 1000 µm aufweist.

12. Verwendung des Polyelektrolytzements nach einem der Ansprüche 1 bis 5 und/oder eines Granulats nach einem der Ansprüche 10 bis 11 in einem Dentalmaterial.

13. Behältnis enthaltend einen Polyelektrolytzement nach einem der Ansprüche 1 bis 5 und/oder ein Granulat nach einem der Ansprüche 10 bis 11.

14. Applikationsvorrichtung enthaltend einen Polyelektrolytzement nach einem der Ansprüche 1 bis 5 und/oder ein Granulat nach einem der Ansprüche 10 bis 11.

## Claims

1. One-component or multicomponent polyelectrolyte cement, containing at least two reaction partners:
(a) at least one compound which releases metal cations, and
(b) one or more polyelectrolytes which can be converted into the solid state, at least one of the polyelectrolytes being at least partially water-soluble, with at least a proportion of the reaction partners (a) and/or (b) being at least partially covered with an organic surface-coating agent, the organic surface-coating agent being a film-forming material and being present in a quantity of from 0.01 to 3% by weight, based on the total weight of the surface-coated material.

2. Polyelectrolyte cement according to Claim 1, in which the two reaction partners (a) and (b) are at least partially within the same component of the polyelectrolyte cement.

3. Polyelectrolyte cement according to any of the preceding claims, in which at least a proportion of the reaction partners (a) and/or (b) present in the same component is covered with an organic surface-coating agent.

4. Polyelectrolyte cement according to any of the preceding claims, in which the organic surface-coating agent is at least partially acid-soluble.

5. Polyelectrolyte cement according to any of the preceding claims, in which at least a proportion of the constituents which are present in solid form is in pulverulent, granulated and/or tableted form.

6. Process for producing a polyelectrolyte cement according to one of the preceding claims in granule form, in which at least a proportion of one or more polyelectrolytes which can be converted into the solid state and are at least partially water-soluble serves as the main granulating agent, and the granulating agent at least partially covers the surface of a constituent which is to be granulated.

7. Process according to Claim 6, which process is carried out as a wet granulation with a solvent or a solvent mixture which dissolves at least a proportion of the reaction partner (b) without triggering a reaction between (a) and (b), and which is removed after the granulation.

8. Process according to either of Claims 6 and 7, in which the solvents used for the wet granulation have polar properties.

9. Process according to Claim 6, in which a dry granulation is carried out.

10. Granules obtainable by a process according to Claims 6 to 9.

11. Granules according to Claim 10 with a mean grain size of between 10 and 1000 µm.

12. Use of the polyelectrolyte cement according to any of Claims 1 to 5 and/or of granules according to either of Claims 10 and 11 in a dental material.

13. Vessel containing a polyelectrolyte cement according to any of Claims 1 to 5 and/or granules according to either of Claims 10 and 11.

14. Applicator containing a polyelectrolyte cement according to any of Claims 1 to 5 and/or granules according to either of Claims 10 and 11.

## Revendications

1. Ciment polyélectrolytique à un ou plusieurs composants, contenant au moins deux partenaires de réaction :
(a) au moins un composé libérant des cations métalliques et
(b) un ou plusieurs polyélectrolytes transformables à l'état solide, au moins un des polyélectrolytes étant au moins partiellement soluble dans l'eau,
au moins une partie des partenaires de réaction (a) et/ou (b) étant partiellement revêtue d'un agent de revêtement de surface organique, l'agent de revêtement de surface organique étant un matériau formant un film et se trouvant en une quantité de 0,01 à 3% en poids par rapport au poids total du matériau à surface revêtue.

2. Ciment polyélectrolytique selon la revendication 1, les deux partenaires de réaction (a) et (b) se trouvant au moins partiellement dans le même composant du ciment polyélectrolytique.

3. Ciment polyélectrolytique selon l'une quelconque des revendications précédentes, au moins une partie des partenaires de réaction (a) et/ou (b) se trouvant dans le même composant étant revêtue d'un agent de revêtement de surface organique.

4. Ciment polyélectrolytique selon l'une quelconque des revendications précédentes, l'agent de revêtement de surface organique étant au moins partiellement soluble dans les acides.

5. Ciment polyélectrolytique selon l'une quelconque des revendications précédentes, au moins une partie des constituants se trouvant à l'état solide se trouvant sous une forme poudreuse, granulée et/ou comprimée.

6. Procédé pour la préparation d'un ciment polyélectrolytique selon l'une quelconque des revendications précédentes sous forme de granulat, au moins une partie d'un ou de plusieurs polyélectrolytiques transformables à l'état solide, qui sont au moins partiellement solubles dans l'eau, servant d'agent de granulation essentiel et l'agent de granulation recouvrant au moins partiellement la surface d'un des constituants à granuler.

7. Procédé selon la revendication 6, le procédé étant réalisé en tant que granulation par voie humide avec un solvant ou un mélange de solvants, qui dissout au moins une partie des partenaires de réaction (b), sans déclencher une réaction entre (a) et (b) et qui est éliminé après la réaction.

8. Procédé selon l'une quelconque des revendications 6 et 7, les solvants utilisés pour la granulation par voie humide présentant des propriétés polaires.

9. Procédé selon la revendication 6, une granulation par voie sèche étant réalisée.

10. Granulat pouvant être obtenu par un procédé selon les revendications 6 à 9.

11. Granulat selon la revendication 10, qui présente une grosseur moyenne des granules entre 10 et 1000 µm.

12. Utilisation du ciment polyélectrolytique selon l'une quelconque des revendications 1 à 5 et/ou d'un granulat selon l'une quelconque des revendications 10 à 11 dans un matériau dentaire.

13. Récipient contenant un ciment polyélectrolytique selon l'une quelconque des revendications 1 à 5 et/ou un granulat selon l'une quelconque des revendications 10 et 11.

14. Dispositif d'application contenant un ciment polyélectrolytique selon l'une quelconque des revendications 1 à 5 et/ou un granulat selon l'une quelconque des revendications 10 et 11.
